# FASCICULE DE BREVET EUROPEEN

(11) **EP 2 646 565 B1**
(45) Date de publication et mention de la délivrance du brevet: **26.07.2017**
(21) Numéro de dépôt: 11797269.5
(22) Date de dépôt: 30.11.2011
(51) Int. Cl.: C12Q 1/04, C12Q 1/06, C12Q 1/18, G01N 33/543, G01N 33/569

(54) **PROCEDE DE DETECTION ET DE QUANTIFICATION DE MICROORGANISMES**
VERFAHREN ZUM NACHWEIS UND ZUR QUANTIFIZIERUNG VON MIKROORGANISMEN
METHOD FOR DETECTING AND QUANTIFYING MICROORGANISMS

(30) Priorité: 01.12.2010 FR 1059983
(43) Date de publication de la demande: 09.10.2013
(73) Titulaire: Commissariat à l'Énergie Atomique et aux Énergies Alternatives, 75015 Paris (FR); Centre National de la Recherche Scientifique, 75794 Paris Cedex 16 (FR); Université Joseph Fourier, 38041 Grenoble Cedex 9 (FR)
(72) Inventeur: ROUPIOZ, Yoann, 38570 Theys (FR); CALEMCZUK, Roberto, 38000 Grenoble (FR); VERNET, Thierry, 38000 Grenoble (FR); LIVACHE, Thierry, 38560 Jarrie (FR); BOUGUELIA, Sihem, 38000 Grenoble (FR); DURMORT, Claire, 38950 Quaix En Chartreuse (FR)
(74) Mandataire: Gevers & Orès
(86) Numéro de dépôt international: PCT/IB2011/055384
(87) Numéro de publication internationale: WO 2012/073202

(56) Documents cités:
- WO-A1-2008/106048
- US-A1- 2008 096 241
- TAYLOR A D ET AL: "Quantitative and simultaneous detection of four foodborne bacterial pathogens with a multi-channel SPR sensor", BIOSENSORS AND BIOELECTRONICS, ELSEVIER BV, NL, vol. 22, no. 5, 15 décembre 2006 (2006-12-15), pages 752-758, XP024961562, ISSN: 0956-5663, DOI: 10.1016/J.BIOS.2006.03.012 [extrait le 2006-12-15] cité dans la demande

## Description

### Domaine de l'invention

La présente invention concerne un procédé de détection et de quantification de microorganismes dans un échantillon.

### Arrière-plan de l'invention

La détection de microorganismes vivants dans certains échantillons est d'une importance cruciale, notamment dans le domaine médical et l'industrie agro-alimentaire.

La méthode standard de détection reste la culture microbienne qui requiert souvent plusieurs jours pour l'obtention d'un nombre de microorganismes suffisant pour identifier le germe recherché. Dans le cas d'une recherche à partir d'un échantillon issu de patient, ce délai avant l'obtention de résultats implique souvent une administration antibiotique préventive à large spectre au patient. Pour les bactéries pathogènes, l'augmentation du nombre de souches résistantes aux antibiotiques est donc une conséquence possible de ce protocole de médication qui ne cible pas une bactérie particulière mais une multitude d'autres espèces bactériennes.

Le diagnostic bactériologique se fait classiquement en deux temps :
- une première phase de croissance, généralement sur milieu solide, des bactéries pour en avoir un nombre suffisant et isoler la bactérie suspectée ; à ce stade, l'étude sur milieu de culture solide, de la forme des colonies permet une identification préliminaire des bactéries ; en outre cette phase est fréquemment couplée à une détection non spécifique des bactéries, par culture en milieu liquide basée sur l'acidification du milieu de culture ou des variations des propriétés optiques du milieu, par exemple par diffusion de la lumière, ce qui donne une idée sur l'éventuelle présence de bactéries dans l'échantillon ;
- une deuxième phase d'identification de la bactérie par croissance en milieu sélectif, puis d'étude des caractères biochimiques et immunologiques, suivant une clé dichotomique, qui va des caractères les plus vastes aux plus pointus, pour aboutir à une espèce bactérienne particulière.

Dans les applications médicales, une étape supplémentaire avant le traitement thérapeutique est souvent nécessaire, elle consiste à réaliser un antibiogramme afin de tester le niveau de résistance de la bactérie vis-à-vis des différents antibiotiques de la pharmacopée.

Cette méthode nécessite au moins 24h de croissance et, pour certaines espèces bactérienne, jusqu'à plusieurs jours de culture avant identification visuelle. Dans tous les cas la durée de l'analyse est cruciale, et les étapes d'enrichissement puis d'analyse sont très longues (un à plusieurs jours).

Afin de réduire ce délai, des méthodes *in vitro* de détection plus rapides de la croissance bactérienne ont été développées. Cependant, toutes ces approches sont basées sur des systèmes multi-étapes impliquant d'abord une phase de culture pour augmenter le nombre de bactéries présentes dans l'échantillon, puis, au moins une phase d'identification et de caractérisation.

Des automates de suivi de la croissance bactérienne ont été récemment introduits sur le marché, par exemple, le BACTEC 9000 de Becton-Dickinson et BacT/ALTER de BioMérieux. Ces automates font appel au dosage de CO₂ dégagé lors de la prolifération bactérienne. L'utilisation de ces automates pour hémoculture a permis d'augmenter la sensibilité des tests en particulier lors d'hémoculture et par conséquent de diminuer les délais de réponse (Lamy et al. (2005) Biotribune 16:37-39 ; Croizet et al. (2007) Spectra Biologie 160:45-51). Ces automates ne permettent toutefois pas d'identifier spécifiquement les genres et espèces de bactéries.

Plus récemment, la société Accelr8 a mis sur le marché un dispositif de concentration microfluidique qui adsorbe, de façon non spécifique, les bactéries sur une surface de polymère chimiquement fonctionnalisée. Dans ce cas, la détection de la croissance de microorganisme précède leur identification. En effet, les bactéries sont d'abord multipliées sur un support puis identifiées grâce à un procédé d'immunofluorescence suivi d'une analyse par microscopie. Il est à noter que ce procédé reste relativement laborieux puisque les deux étapes de culture puis de détection sont réalisées successivement.

Des procédés assez proches impliquant un système d'impulsions électriques pour concentrer les spores de *Bacillus subtilis* afin de les détecter par un système optique associé ont été développés par Zourob et al. (2005) Lab Chip 5:1350-1365. Ce procédé n'implique pas de culture et détecte directement les spores présentes dans un échantillon. Il nécessite cependant l'utilisation de microsystèmes assez complexes.

Des techniques utilisant des procédés de détection électrochimiques ont également été proposées. Les plus performantes sont basées sur des principes impédimétriques (Yang et al. (2004) Anal. Chem.76:1107-13 ; Huang et al. (2010) Biosensors & Bioelectronics 25:1204-11). Ces procédés impliquent souvent l'utilisation d'un médiateur redox utilisé comme sonde. Dans ce cas, le milieu de mesure doit être supplémenté en médiateur, typiquement l'hexacyanoferrate, utilisé à des concentrations de l'ordre de 1 à 10 mM. Cela reste un inconvénient majeur puisque l'effet de concentrations relativement élevées (mM) de ce type de composés sur les microorganismes à détecter reste mal connu et peut être source d'interférences avec le milieu biologique.

Les techniques optiques sans traceur peuvent en principe s'affranchir de l'ajout de composés exogènes et sont donc plus directes. La technique de résonance plasmonique de surface (SPR) a été appliquée à la détection de lysats bactériens par Taylor et al. (2006) Biosensors and Bioelectronics 22:752-758. Dans ce cas, la détection est précédée par une lyse bactérienne puis, le signal est amplifié par une liaison avec un anticorps secondaire spécifique. Des limites de détection situées entre 10⁴ et 10⁶ bactéries/ml sont atteintes. Ce procédé nécessite donc, soit des échantillons très contaminés soit une culture préalable pour atteindre ces seuils. De plus, étant donné que la détection se réalise sur lysat bactérien, la méthode n'est pas adaptée à l'indentification de bactéries vivantes. De même, dans la demande WO 2008/106048 qui propose une détection multiplexe de microorganismes par SPR, des lysats bactériens sont utilisés comme analytes.

La demande US 2008/0096241 divulgue une méthode pour déterminer la sensibilité d'un microorganisme à un antibiotique, à l'aide d'un dispositif de SPR. Selon cette méthode, les bactéries dont on souhaite connaître la sensibilité à un antibiotique sont adsorbées à une surface adaptée à la SPR et fonctionnalisée par un ligand non spécifique, avant d'ajouter l'antibiotique à tester; pendant ces étape, le signal SPR est mesuré, reflétant la présence et l'état des bactéries adsorbées. La comparaison des courbes obtenues avec l'échantillon à tester et un échantillon contrôle dont on connaît la sensibilité à l'antibiotique testé donne l'information recherchée.

La demande WO 2008/106048 concerne un appareil pour mesurer l'activité cellulaire induite par un ligand, l'appareil comprenant un biocapteur optique ayant une surface de contact, qui comprend une zone d'agents de compatibilité, connectée de manière directe ou indirecte à la surface du biocapteur, ainsi qu'une zone cellulaire, comprenant au moins une cellule associée à au moins un agent de compatibilité. Ce dispositif n'a pas pour objet de détecter des cellules dans un échantillon, et il ne permet pas de mesurer simultanément le signal provenant d'un ligand spécifique du microorganismes et celui provenant d'un capteur non spécifique.

Enfin, d'autres travaux ont été rapportés, qui permettent de détecter la germination des spores d*'Aspergillus niger* et de *Candida albicans* à l'aide de micro-leviers (Nugaeva et al. (2007) Microsc. Microanal. 13:13-17 ; Nugaeva et al. (2005) Biasensors and Bioelectronics 21:89-856). Ce type de détection se déroule dans l'air, en chambre humide et présente l'inconvénient de ne pas pouvoir être réalisé en milieu liquide. Par ailleurs, elle n'est applicable qu'à la détection de la germination de spores fongiques, préalablement capturées.

### Résumé de l'invention

La présente invention découle de la découverte, par les inventeurs, que le couplage de la culture d'un microorganisme et de la mesure d'un signal différentiel généré par la fixation spécifique du microorganisme par un ligand, c'est-à-dire que la culture et la mesure du signal différentiel se font de manière simultanée, permettait d'abaisser le seuil de détection du microorganisme et de diminuer significativement le temps d'analyse, par rapport aux techniques habituelles.

En conséquence, la présente invention concerne un procédé de détection d'au moins un microorganisme, présent dans un échantillon, comprenant :
- la culture en milieu liquide de l'échantillon en présence d'au moins un ligand spécifique du microorganisme et d'au moins un capteur non spécifique du microorganisme, la liaison du microorganisme sur le ligand produisant un premier signal mesurable et la liaison du microorganisme sur le capteur produisant un deuxième signal mesurable ;
- la détermination de valeurs du premier et du deuxième signal pour au moins une durée de culture ;
dans lequel on déduit que l'échantillon comprend le microorganisme lorsque les valeurs du premier signal et du deuxième signal pour une même durée de culture sont différentes.

Dans un mode de réalisation particulier du procédé selon l'invention, lorsque l'échantillon comprend un microorganisme, on détermine en outre la quantité de microorganismes présents dans l'échantillon au début de la culture à partir de l'évolution de la valeur du premier signal en fonction de la durée de culture.

Dans un autre mode de réalisation du procédé selon l'invention, lorsque l'échantillon comprend un microorganisme, on détermine la sensibilité du microorganisme à au moins un antimicrobien à partir de l'évolution de la valeur du premier signal en fonction de la durée de culture après introduction de l'antimicrobien dans la culture.

La présente invention concerne également un dispositif adapté à la mise en oeuvre d'un procédé tel que défini ci-dessus, comprenant au moins une chambre adaptée pour la culture en milieu liquide d'un microorganisme, la chambre comprenant au moins un ligand spécifique du microorganisme et au moins un capteur non spécifique du microorganisme, la liaison du microorganisme sur le ligand produisant un premier signal mesurable et la liaison du microorganisme sur le capteur produisant un deuxième signal mesurable, le ligand et le capteur étant fixés sur un support de manière à être en contact avec le milieu liquide à étudier.

### Description détaillée de l'invention

Comme on l'entend ici, un microorganisme est de préférence un organisme procaryote ou eucaryote, unicellulaire ou pluricellulaire. Toutefois, lorsque le microorganisme est pluricellulaire, on préfère que les cellules constituant le microorganisme pluricellulaire soient homogènes en terme de différentiation, c'est-à-dire que le microorganisme ne comprenne pas des cellules ayant différentes spécialisations. De préférence, le microorganisme selon l'invention est vivant, c'est-à-dire qu'il est capable de se multiplier. Par ailleurs, le microorganisme selon l'invention est de préférence unicellulaire. A ce titre, le microorganisme selon l'invention peut être une forme unicellulaire d'un organisme pluricellulaire selon son stade de développement ou de reproduction. En outre, un microorganisme selon l'invention peut également être constitué de cellules isolées ou de fragments de tissus isolés d'un métazoaire. Ainsi, le microorganisme selon l'invention peut également être une cellule de mammifère, notamment humaine, tel qu'une cellule tumorale ou une cellule sanguine, par exemple un lymphocyte ou une cellule mononucléée du sang périphérique. De préférence, le microorganisme selon l'invention est donc un microorganisme, en particulier unicellulaire, sélectionné dans le groupe constitué d'une bactérie, d'un champignon, d'une levure, d'une algue, d'un protozoaire, et d'une cellule isolée de métazoaire. De manière particulièrement préférée, le microorganisme selon l'invention est une bactérie, en particulier du genre *Streptococcus* ou *Escherichia.*

L'échantillon selon l'invention peut être de tout type, dans la mesure où il peut comprendre un microorganisme. De préférence, l'échantillon selon l'invention est sélectionné dans le groupe constitué d'un échantillon biologique, d'un échantillon alimentaire, d'un échantillon d'eau, notamment d'eau usée, d'eau douce ou de mer, d'un échantillon de sol, d'un échantillon de boue et d'un échantillon d'air. Les échantillons biologiques selon l'invention proviennent d'organismes vivants ou ayant été vivants, notamment d'animaux ou de végétaux. Les échantillons provenant d'animaux, notamment de mammifère, peuvent être liquides ou solides, et comprennent, notamment, le sang, le plasma, le sérum, le liquide céphalorachidien, l'urine, les selles, le liquide synovial, le sperme, les sécrétions vaginales, les sécrétions buccales, des prélèvements respiratoires, provenant en particulier des poumons, du nez, ou de la gorge, du pus, des liquides d'ascite, ou des prélèvements de sérosités cutanées ou conjonctivales. De préférence, les échantillons alimentaires selon l'invention proviennent notamment d'aliments, qui peuvent être crus, cuits ou cuisinés, d'ingrédients alimentaires, d'épices ou de plats cuisinés. De préférence également, l'échantillon selon l'invention n'est pas purifié ou concentré avant d'être mis en culture selon l'invention.

Comme cela apparaîtra de manière claire à l'homme du métier, la culture en milieu liquide selon l'invention est conduite de manière à obtenir une multiplication du microorganisme éventuellement présent dans l'échantillon mis en culture et que l'on cherche à détecter. Les techniques et les conditions de culture de microorganismes en milieu liquide sont bien connues de l'homme du métier, qui sait notamment définir, pour chaque microorganisme, le milieu nutritif adéquat, la température de croissance optimale, par exemple 37°C pour de nombreuses bactéries pathogènes des mammifères, ainsi que l'atmosphère nécessaire à la multiplication d'un microorganisme selon l'invention. Par ailleurs, des milieux de culture faiblement sélectifs, c'est-à-dire permettant la croissance d'un grand nombre de microorganismes de types différents, seront préférentiellement utilisés. La durée de culture est également adaptable pour chaque microorganisme, en fonction de sa vitesse de croissance ou de son temps de génération, c'est-à-dire le temps nécessaire à la division du microorganisme en deux microorganismes filles. De préférence, la durée de culture selon l'invention est supérieure ou égale au temps nécessaire à la production d'au moins deux générations successives de microorganismes filles qui permet donc au moins un quadruplement, c'est-à-dire une multiplication par 4, du nombre de microorganismes à détecter. L'homme du métier comprendra aisément qu'il n'est pas nécessaire d'observer effectivement le quadruplement du nombre de microorganismes en culture, dans la mesure où le microorganisme à détecter peut être absent de l'échantillon selon l'invention, mais que la durée de culture correspond au minimum à celle permettant d'obtenir un quadruplement du nombre de microorganismes dans une culture qui contient effectivement le microorganisme et qui est conduite dans les même conditions que celle du procédé de l'invention. Ainsi, on préfère également que la durée de culture selon l'invention soit au moins égale à deux fois le temps de génération ou le temps de doublement du microorganisme à détecter. Comme l'homme du métier le comprendra bien, le temps de génération ou de doublement selon l'invention est celui qui peut être obtenu dans les conditions de culture selon l'invention. Généralement, la durée de culture sera inférieure à 72 h, 48 h ou 24 h. En outre, la culture pourra être stoppée dès que l'information recherchée, détection, détermination de la quantité, ou sensibilité aux antimicrobiens, aura pu être obtenue.

Avantageusement, le procédé de l'invention est sensible et permet de détecter la présence de microorganismes dans des échantillons contenant des concentrations faibles de microorganisme, par exemple égales à 1 ou inférieures à 10, 10² ou 10³ microorganismes/ml, qui sont habituellement indétectables directement, notamment à l'aide d'un biocapteur. Ainsi, on préfère que le procédé de l'invention soit appliqué à des échantillons que l'on suspecte de contenir un microorganisme à détecter à une concentration inférieure ou égale à 10⁶, 10⁵, 10⁴, 10³, 10², ou 10 microorganismes/ml, ou égale à 1 microorganisme/ml.

Le ligand selon l'invention est de tout type permettant de se lier spécifiquement à un microorganisme ou à plusieurs microorganismes apparentés. Il peut notamment s'agir :
- d'un récepteur naturel du microorganisme, tel qu'un sucre polysaccharidique ou un lipide complexe, c'est-à-dire un lipide comprenant au moins un groupement alipidique, notamment de type protéique, glucidique, phosphoré, ou azoté ; d'une protéine ou d'une glycoprotéine, telle que le plasminogène par exemple, qui se lie à plusieurs espèces bactériennes du genre *Streptococcus* ; de bactériophages ou de virus entiers, éventuellement inactivés, de fragments de bactériophages ou de virus ou de protéines de bactériophages ou de virus ;
- d'immunoglobulines, telles que les anticorps et leurs fragments comprenant la partie variable, ciblant spécifiquement un antigène, ou constante, qui peut être liée par les bactéries du genre *Staphylococcus* qui comprennent la protéine A, ou les fragments de récepteurs de cellules T (TCR), comprenant notamment la partie variable ;
- de composés synthétiques, notamment :
   - des petites molécules organiques comprenant en particulier de 1 à 100 atomes de carbone,
   - des composés de nature peptidique, tels que des scFv (*single chain variable fragment*),
   - des composés de nature polynucléotidique, tels que les aptamères, ou
   - des composés de nature polysaccharidique ;
- des broyats, lysats ou extraits d'organismes ou de tissus vivants ;
- des matériaux synthétiques présentant une surface d'adsorption de microorganismes ; ou
- de mélanges comprenant deux, ou plus, des ligands définis ci-dessus.

Ainsi, le ligand selon l'invention est de préférence sélectionné dans le groupe constitué d'un anticorps, d'un fragment d'anticorps, d'un scFv, d'un aptamère, d'une protéine ou d'une glycoprotéine, de bactériophages entiers, éventuellement inactivés, de fragments de bactériophages, et de protéines de bactériophages.

Le capteur selon l'invention sert de témoin négatif. En conséquence, il est de préférence d'une nature semblable à celle du ligand. Par ailleurs, le capteur selon l'invention présente moins d'affinité pour le microorganisme à détecter que le ligand, c'est-à-dire que selon l'invention il présente de préférence une affinité pour le microorganisme inférieure au moins 10 fois, notamment inférieure au moins 100 fois, 1000 fois, 10 000 fois, 100 000 ou 1 000 000 fois à celle du ligand pour le microorganisme. En particulier, les affinités du ligand et du capteur pour le microorganisme peuvent être déterminées par la méthode de Scatchard dans les mêmes conditions expérimentales. De manière davantage préférée, le capteur selon l'invention ne présente pas d'affinité pour le microorganisme.

De préférence, on considère selon l'invention que les valeurs du premier signal et du deuxième signal pour une même durée de culture sont différentes lorsque la différence du premier signal et du deuxième signal est supérieure ou égale à deux fois le signal mesuré du bruit de fond. Le signal mesuré du bruit de fond correspond notamment au premier signal mesuré en l'absence de microorganisme.

Comme on l'entend ici, le signal mesurable selon l'invention est directement généré par la fixation ou la liaison d'un composé, notamment un microorganisme, sur le ligand ou le capteur.

En particulier, le signal mesurable selon l'invention ne provient de préférence pas d'un médiateur, telle qu'une sonde d'oxydoréduction, ou d'un marqueur présent dans le milieu, ou ajouté au milieu, autre que le ligand et le capteur selon l'invention. Ainsi, de préférence, le signal mesurable selon l'invention ne provient pas de la fixation additionnelle d'un marqueur spécifique du microorganisme sur un microorganisme déjà fixé par le ligand ou le capteur.

Le signal peut être mesuré par toute technique adaptée à la mesure d'au moins deux signaux simultanément, en particulier directe, et notamment par microscopie, par résonance plasmonique de surface, par miroirs résonnants, par mesure d'impédance, par un microsystème électromécanique (MEMS), tel que des microbalances à quartz ou des poutres flexibles, par mesure d'absorption de lumière, en particulier ultraviolette ou visible, ou encore par mesure de fluorescence, notamment si le microorganisme est lui même fluorescent, qui sont bien connues de l'homme du métier. A ce titre, comme cela apparaîtra clairement à l'homme du métier, le marqueur défini ci-dessus ne désigne pas un microorganisme selon l'invention ; ainsi le signal selon l'invention peut provenir du microorganisme lorsqu'il est fixé par le ligand ou le capteur. La résonance plasmonique de surface est particulièrement préférée selon l'invention.

De préférence, le ligand et le capteur sont fixés sur un support. Plus préférablement, le support permet la transduction du signal produit par la liaison d'un composé au ligand et/ou au capteur, notamment de sorte que le signal puisse être mesuré par les techniques mentionnées ci-dessus. De tels supports sont bien connus de l'homme du métier et comprennent, notamment, des substrats transparents recouverts de surfaces métalliques, continues ou discontinues, adaptés à la mesure par résonance plasmonique de surface. Ainsi, dans un mode de réalisation préféré de l'invention, le ligand et le capteur sont fixés sur un support, identique ou différent, qui est lui-même constitutif d'une biopuce. Par ailleurs, le ligand et le capteur, le ou les supports, ou le dispositif, selon l'invention, peuvent être compris dans un récipient destiné à recueillir des liquides susceptibles de contenir des microorganismes, ou destiné à cultiver des microorganismes, tels qu'un flacon d'hémoculture ou une poche de stomacher par exemple.

De préférence également, le signal est mesuré en temps réel, notamment sans qu'il soit nécessaire d'effectuer des prélèvements de la culture pour mesurer le signal. Le signal mesuré est de préférence enregistré, par exemple sous la forme de courbe portant l'intensité du signal mesuré en fonction du temps. Il est également possible d'enregistrer des images du support sur lequel sont fixés le ligand et le capteur selon l'invention afin de déterminer le degré, ou le niveau, d'occupation du ligand et du capteur par le microorganisme.

Dans des modes de réalisation particuliers de l'invention, le procédé selon l'invention permet de détecter la présence de plusieurs microorganismes différents, de déterminer la quantité de différents microorganismes présents dans l'échantillon, ou de déterminer la sensibilité de différents microorganismes à un ou plusieurs antimicrobiens, il suffit alors que plusieurs ligands respectivement spécifiques des différents microorganismes soient utilisés. Un tel procédé est alors dit multiplexe.

Comme on l'entend ici, le terme « antimicrobien » se réfère à tout composé microbicide, inhibant la croissance du microorganisme, ou réduisant sa prolifération, notamment à tout composé antibiotique, bactéricide ou bactériostatique, tel que l'érythromycine. Par ailleurs, le terme « antimicrobien » se réfère également, notamment lorsque le microorganisme selon l'invention est une cellule tumorale, à tout composé anticancéreux, notamment chimiothérapeutique, destiné à détruire le microorganisme ou à réduire sa prolifération.

De préférence, lorsqu'on détermine la sensibilité du microorganisme à au moins un antimicrobien selon l'invention, l'évolution de la valeur du premier signal en fonction de la durée de culture provient, en partie ou en totalité, d'une variation du temps nécessaire à la division d'un microorganisme, c'est-à-dire du temps de génération, et ainsi de l'évolution au cours du temps du nombre de microorganismes qui viennent se lier sur le premier ligand. Comme l'homme du métier le comprend bien, si le temps de génération du microorganisme s'accroit significativement, la variation du nombre de microorganismes sera plus faible que celle obtenue en absence de l'antimicrobien. Ainsi, de manière préférée, l'évolution de la valeur du premier signal n'est pas due à la destruction par l'antimicrobien des microorganismes fixés sur le premier ligand selon l'invention.

### Description des figures

Figures 1, 2 et 3

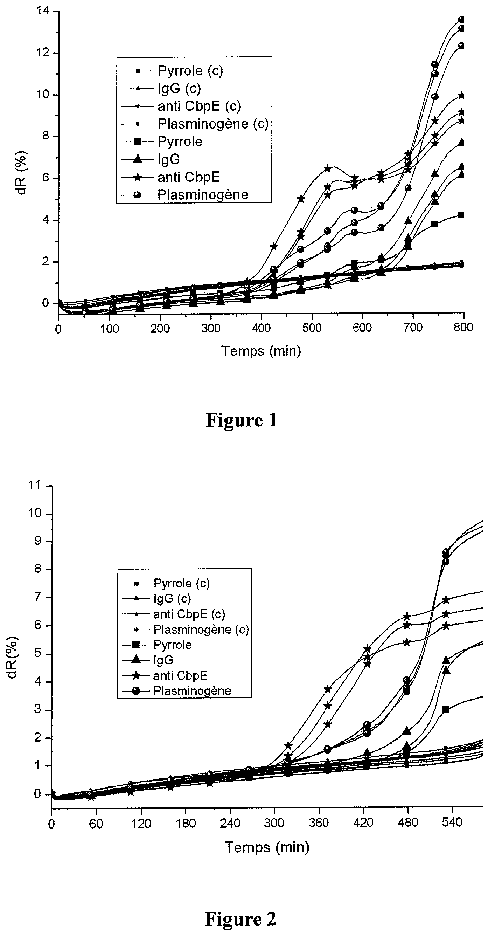

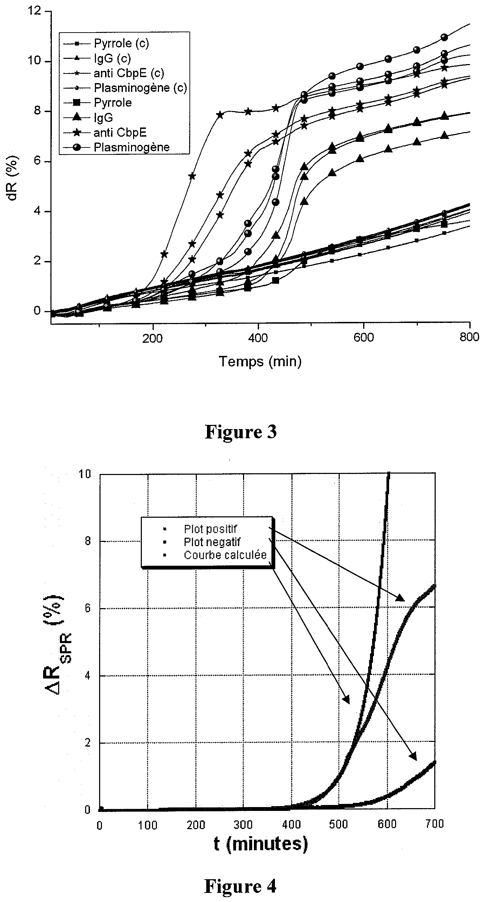

   Les figures 1 à 3 sont issues de trois expériences indépendantes et représentent la variation de réflectivité (dR, axe des ordonnées, en %) mesurée par imagerie de résonance plasmonique de surface, en fonction de la durée de culture (axe des abscisses, temps en min), d'une biopuce fonctionnalisée à l'aide (i) d'anticorps anti-CbpE (grandes étoiles, triplicat) dirigés contre *Streptococcus pneumoniae,* (ii) de plasminogène (Plg) (grands cercles, triplicat), (iii) d'IgG non spécifiques de *S. pneumoniae* (grands triangles, triplicat) et (iv) de pyrrole uniquement (grand carrés), et mise en présence d'une culture de *S. pneumoniae* souche R6 à la concentration initiale de 10² bactéries/ml (Figure 1), 10³ bactéries/ml (Figure 2) et 10⁴ bactéries/ml (Figure 3), dans un volume d'échantillon de 500 µL (soient respectivement 50, 500 et 5000 bactéries). Par ailleurs, à titre de témoins, et notées (c), les courbes obtenues à l'aide de la même biopuce mise en présence d'un milieu de culture non ensemencé sont également présentées.
Figure 4
   La figure 4 représente la variation de réflectivité ΔR_{SPR}, axe des ordonnées, en %), mesurée par imagerie de plasmons de surface, d'une biopuce fonctionnalisée à l'aide d'anticorps anti-CbpE dirigés contre *Streptococcus pneumoniae* (plot positif) et de pyrrole uniquement (plot négatif), en présence d'une culture de *S*. *pneumoniae,* en fonction de la durée de culture (axe des abscisses, t en min), ainsi qu'une courbe modélisant la réflectivité du plot fonctionnalisée à l'aide d'anticorps anti-CbpE (courbe calculée).
Figure 5

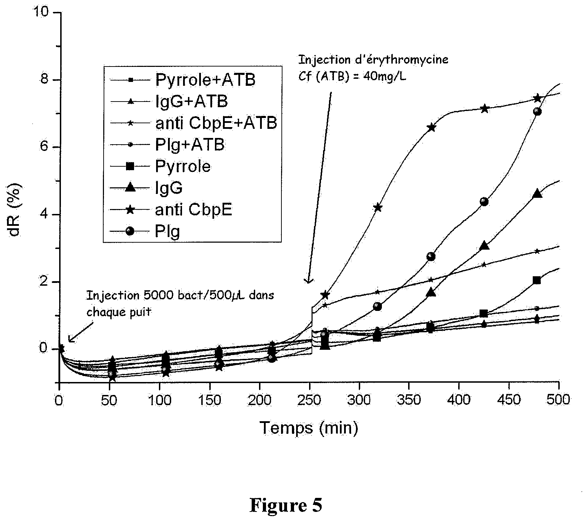

   La figure 5 représente la variation de réflectivité (dR, axe des ordonnées, en %) d'une biopuce fonctionnalisée à l'aide d'anticorps anti-CbpE dirigés contre *Streptococcus pneumoniae,* de plasminogène (Plg), d'IgG non spécifiques de *S. pneumoniae,* et de pyrrole uniquement, mise en présence d'une culture de *S. pneumoniae* souche R6 à la concentration initiale de 10⁴ bactéries/ml dans deux cuves en parallèle, dans lesquelles est respectivement ajouté au temps t = 250 min, (i) de l'érythromycine à une concentration finale de 40 mg/ml diluée dans l'éthanol à la concentration finale de 0,08%, (+ ATB), (ii) de l'éthanol (EtOH) à la concentration finale de 0,08%.

### EXEMPLES

### Exemple 1 : détection de microorganismes dans un échantillon

### Construction d'une biopuce

Une biopuce à protéines a été préparée en utilisant la méthode d'éléctro-polymérisation des protéines sur un prisme à surface dorée (utilisé comme une électrode de travail), comme cela est décrit par Grosjean et al. (2005) Analytical Biochemistry 347:193-200, à partir d'un conjugué protéine-pyrrole en présence de pyrrole libre. Brièvement, l'électro-polymérisation du pyrrole libre et des protéines couplées au pyrrole-NHS est faite par un cône de pipette contenant une tige en platine servant de contre électrode ; la polymérisation est effectuée par des pulses électriques rapides de 100ms (2.4 V) entre l'électrode de travail et la contre-électrode, comme cela est notamment décrit par Guédon et al. (2000) Anal. Chem. 72:6003-6009. Chaque espèce protéique a été déposée sous forme de triplicats sur la surface dorée du prisme afin d'estimer la reproductibilité du processus.

Les ligands utilisés sont les suivants :
- Ligands reconnaissant *Streptoccocus pneumoniae*
   (i) anticorps anti-CbpE préparés selon Attali et al. (2008) Infect. Immun. 76:466-76
   (ii) plasminogène humain (Sigma Aldrich).
- Capteurs pour les témoins négatifs :
   (iii) IgG de lapin purifiées (Sigma Aldrich) ou IgG monoclonaux anti-toxine botulique ;
   (iv) Pyrrole (Tokyo kasei, TCI)

Tous ces produits ont été couplés au pyrrole puis déposés sous forme de plots sur le prisme doré selon un plan de dépôt adapté à la forme des cuves permettant la culture des microorganismes. Chaque cuve est munie de 2 compartiments indépendants pouvant contenir deux échantillons différents dont un témoin.

### Préparation des cultures de bactéries

Les pneumocoques souche R6 ont été cultivés dans du milieu de culture Todd Hewitt (TH de *Mast Diagnostic*). La culture a été arrêtée pendant la phase de croissance optimale des bactéries, c'est à dire à une densité optique mesurée à 600 nm (DO₆₀₀) de 0,4, ce qui correspond à une concentration bactérienne d'environ 10⁸ bactéries/ml. Des dilutions successives ont été réalisées en milieu TH afin d'obtenir des concentrations de 10² à 10⁴ bactéries/ml.

### Mesure en imagerie de résonance plasmonique de surface (SPRi) :

Les mesures en imagerie SPR ont été réalisées à l'aide du système SPRi-Lab de Genoptics (Orsay, France).

La biopuce a été préalablement bloquée à l'aide de tampon PBS comprenant 1% de sérum albumine bovine (BSA) pendant 15 minutes. 0,5 millilitre d'échantillon contenant éventuellement des bactéries sont introduits dans le compartiment « échantillon », alors que du milieu de culture dépourvu de pneumocoques est introduit dans le compartiment « témoins ». Le système est placé dans l'enceinte de l'appareil SPRi chauffée à 37°C. La cuve est bouchée afin d'éviter l'évaporation du milieu de culture pendant la croissance réalisée en l'absence d'agitation.

En parallèle, des témoins de croissance ont été réalisés. Plusieurs tube contenant 1 ml de la culture à 10³ bactérie/ml ont été préparés et incubés dans une étuve à 37°C. La DO₆₀₀ a été mesurée toutes les heures, afin de suivre l'évolution de la croissance bactérienne en fonction du temps et la comparer avec les courbes de croissance en SPRi. Les bactéries ont été cultivées pendant 16 heures.

**Tableau 1 : Suivi de la croissance par mesure de la densité optique à 600 nm**

| Durée de culture | DO |
|---|---|
| 1 heure | 0,08 |
| 2 heures | 0,13 |
| 3 heures | 0,15 |
| 4 heures | 0,17 |
| 5 heures | 0,19 |
| 6 heures | 0,25 |
| 7 heures | 0,46 |

### Etude de la sélectivité

Après injection de 500 bactéries, on observe sur la **Figure 2** que les premiers signaux, liés à la capture des bactéries par les anticorps anti-CbpE ainsi que par le plasminogène, sont détectables après environ 300 minutes. Au delà de 400 minutes, un signal devient visible pour les plots portant les témoins négatifs (accrochages non spécifiques). Les signaux (c) obtenus dans la cuve témoins, sans ensemencement bactérien, restent négatifs

On observe également une croissance bactérienne significative entre 300 et 400 minutes à partir du **Tableau 1.**

L'injection d'une quantité moindre de bactéries (50 bactéries, Figure 1), et d'une quantité supérieure (5000 bactéries, Figure 3), génère également un accroissement de signal visible. Les décalages temporels observés correspondent bien à un temps de génération de l'ordre de 30 minutes.

### Exemple 2 : quantification des bactéries présentes dans l'échantillon de départ

La **Figure 4** présente l'évolution temporelle des variations de la réflectivité mesurée en imagerie SPR (ΔR_{SPR}) observées sur le plot fonctionnalisé par l'anticorps anti-CbpE et sur un plot témoin négatif comprenant uniquement du pyrrole.

On voit qu'après une période de latence d'environ 400 minutes, pendant laquelle l'évolution du signal est masquée par le bruit expérimental, l'augmentation de réflectivité est clairement exponentielle.

A ce titre, la **Figure 4** montre également la courbe représentative de la fonction ΔR_{SPR} = Rₒ 2^{(t/τ)} - Rₒ qui modélise l'évolution de ΔR_{SPR} observée sur le plot fonctionnalisé par l'anticorps anti-CbpE en utilisant pour la valeur de 30 minutes pour τ qui est typique du temps de doublement de population associé à *Streptococcus pneumoniae.* Le facteur multiplicatif Rₒ est proportionnel au nombre de microorganismes présents initialement dans l'échantillon. La détermination de ce facteur permet donc une évaluation quantitative des bactéries présentes initialement dans l'échantillon.

A partir de 550 minutes, on s'écarte de ce régime exponentiel. Cette réduction du rythme de croissance peut être attribuée aux limitations induites par des modifications du milieu de culture, notamment sont appauvrissement susceptible d'affecter significativement la croissance bactérienne, comme le sait l'homme du métier.

La courbe du témoin négatif reste près de zéro jusqu'à 600 minutes. Par la suite, on peut observer un début d'augmentation de ΔR_{SPR} attribuable aux interactions non spécifiques entre *Streptococcus pneumoniae* et la surface du plot témoins.

### Exemple 3 : Inhibition d'une croissance par addition d'antibiotique

La **Figure 5** présente l'impact de l'ajout d'un antibiotique (ABT) (érythromycine, Aldrich) ciblant les pneumocoques, à la concentration finale de 40 mg/ml, et d'éthanol (EtOH) à la concentration finale de 0,08%, sur la réflectivité d'une culture *Streptococcus pneumoniae,* ensemencée à 10³ bactéries/ml, après 250 min de culture.

On observe que l'ajout de l'antibiotique provoque une nette diminution de la pente des courbes reflétant la réflectivité particulièrement marquée pour les plots portant les anticorps anti-CbpE et le plasminogène. La diminution est donc liée à une inhibition de la croissance bactérienne. Par ailleurs, aucune diminution n'est observée lors de l'ajout de solution contrôle à la même concentration d'éthanol, ce qui démontre que l'inhibition de la croissance bactérienne précédemment observée est directement imputable à l'action de l'antibiotique et non pas à un effet de solvant.

Par conséquent, le procédé de quantification de la croissance bactérienne de l'invention est utile pour établir un antibiogramme.

## Revendications

1. Procédé de détection d'au moins un microorganisme présent dans un échantillon, comprenant :
- la culture en milieu liquide de l'échantillon en présence d'au moins un ligand spécifique du microorganisme et d'au moins un capteur non spécifique du microorganisme, la liaison du microorganisme sur le ligand produisant un premier signal mesurable et la liaison du microorganisme sur le capteur produisant un deuxième signal mesurable ;
- la détermination de valeurs du premier et du deuxième signal pour au moins une durée de culture ;
dans lequel on déduit que l'échantillon comprend le microorganisme lorsque les valeurs du premier signal et du deuxième signal pour une même durée de culture sont différentes.

2. Procédé de détection selon la revendication 1, dans lequel la durée de culture est d'au moins deux fois le temps de génération du microorganisme.

3. Procédé de détection selon la revendication 1 ou 2, dans lequel le microorganisme est sélectionné dans le groupe constitué d'une bactérie, d'un champignon, d'une algue, d'un protozoaire, et d'une cellule isolée de métazoaire.

4. Procédé selon l'une des revendications 1 à 3, dans laquelle le ligand est sélectionné dans le groupe constitué d'un anticorps, d'un fragment d'anticorps, d'un scFv, d'un aptamère (ADN, ARN), d'une protéine ou d'une glycoprotéine, de bactériophages ou de virus entiers, de fragments de bactériophages ou de virus, et de protéines de bactériophages ou de virus.

5. Procédé selon l'une des revendications 1 à 4, dans lequel l'échantillon est sélectionné dans le groupe constitué d'un échantillon biologique, d'un échantillon alimentaire, d'un échantillon d'eau, d'un échantillon de sol, et d'un échantillon d'air.

6. Procédé selon l'une des revendications 1 à 5, dans lequel le ligand et/ou le capteur sont fixés sur un support de transduction du signal produit par la liaison d'un composé au ligand et/ou au capteur.

7. Procédé selon l'une des revendications 1 à 6, dans lequel le premier et le deuxième signal sont mesurés en temps réel.

8. Procédé selon l'une des revendications 1 à 7. dans lequel le signal est mesuré par microscopie, par résonance plasmonique de surface, par miroir résonnant, par mesure d'impédance, par microbalance à quartz, par poutre flexible, par mesure d'absorption de lumière, ou par mesure de fluorescence de microorganismes fluorescents.

9. Procédé selon l'une des revendications 1 à 8, dans lequel le signal est mesuré en temps réel par résonance plasmonique de surface.

10. Procédé selon l'une des revendications 1 à 9, dans lequel le signal mesurable selon l'invention ne provient pas d'un médiateur ou d'un marqueur présent dans le milieu, ou ajouté au milieu, autre que le ligand et le capteur selon l'invention.

11. Procédé selon l'une des revendications 1 à 10. dans lequel, lorsque l'échantillon comprend un microorganisme, on détermine en outre la quantité de microorganismes présents dans l'échantillon au début de la culture à partir de l'évolution de la valeur du premier signal en fonction de la durée de culture.

12. Procédé selon l'une des revendications 1 à 11, dans lequel, lorsque l'échantillon comprend un microorganismes, on détermine la sensibilité du microorganisme à au moins un antimicrobien à partir de l'évolution de la valeur du premier signal en fonction de la durée de culture après introduction de antimicrobien dans la culture.

13. Procédé selon l'une des revendications 1 à 11, dans lequel on détermine la présence ou la quantité de plusieurs microorganismes différents dans l'échantillon.

14. Dispositif adapté à la mise en oeuvre d'un procédé tel que défini dans l'une des revendications 1 à 13, comprenant au moins une chambre adaptée pour la culture en milieu liquide d'un microorganisme, la chambre comprenant au moins un ligand spécifique du microorganisme et au moins un capteur non spécifique du microorganismes, la liaison du microorganisme sur le ligand produisant un premier signal mesurable et la liaison du microorganisme sur le capteur produisant un deuxième signal mesurable, le ligand et le capteur étant fixés sur un support de manière à être en contact avec le milieu de culture liquide.

15. Dispositif selon la revendication 14, compris dans un récipient destiné à recueillir des liquides susceptibles de contenir des microorganisms et/ou à cultiver des microorganismes.

## Patentansprüche

1. Verfahren zur Detektion wenigstens eines Mikroorganismus, der in einer Probe vorliegt, umfassend:
- die Kultur der Probe in flüssigem Medium in Gegenwart wenigstens eines spezifischen Liganden des Mikroorganismus und wenigstens eines nicht-spezifischen Sensors des Mikroorganismus, wobei die Bindung des Mikroorganismus an dem Liganden ein erstes messbares Signal produziert, und die Bindung des Mikroorganismus an den Sensor ein zweites messbares Signal produziert;
- die Bestimmung von Werten des ersten und des zweiten Signals für wenigstens eine Kulturdauer;
in dem man den Schluss zieht, dass die Probe den Mikroorganismus umfasst, wenn die Werte des ersten Signals und des zweiten Signals für eine selbe Kulturdauer unterschiedlich sind.

2. Verfahren zur Detektion gemäß Anspruch 1, indem die Kulturdauer wenigstens das zweifache der Generationszeit des Mikroorganismus ist.

3. Verfahren zur Detektion gemäß Anspruch 1 oder 2, in dem der Mikroorganismus aus der Gruppe, bestehend aus einem Bakterium, einem Pilz, einer Alge, einem Protozoon und einer isolierten Metazoonzelle, ausgewählt wird.

4. Verfahren gemäß einem der Ansprüche 1 bis 3, in dem der Ligand aus der Gruppe, bestehend aus einem Antikörper, einem Antikörperfragment, einem scFv, einem Aptamer (DNA, RNA), einem Protein oder einem Glycoprotein, ganzen Bakteriophagen oder Viren, Fragmenten von Bakteriophagen oder Viren und Proteinen von Bakteriophagen oder Viren, ausgewählt wird.

5. Verfahren gemäß einem der Ansprüche 1 bis 4, in dem die Probe aus der Gruppe, bestehend aus einer biologischen Probe, einer Lebensmittelprobe, einer Wasserprobe, einer Bodenprobe und einer Luftprobe, ausgewählt wird.

6. Verfahren gemäß einem der Ansprüche 1 bis 5, in dem der Ligand und/oder der Sensor auf einem Transduktionsträger für das erzeugte Signal durch Bindung einer Verbindung an den Liganden und/oder den Sensor fixiert werden.

7. Verfahren gemäß einem der Ansprüche 1 bis 6, in dem das erste und das zweite Signal in Echtzeit gemessen werden.

8. Verfahren gemäß einem der Ansprüche 1 bis 7, in dem das Signal durch Mikroskopie, durch Oberflächenplasmonresonanz, durch Resonanzspiegel, durch Impedanzmessung, durch Quartzkristall-Mikrowaage, durch flexiblen Träger (poutre felxible), durch Messung der Lichtadsorption oder durch Fluoreszenzmessung von fluoreszierenden Mikroorganismen gemessen wird.

9. Verfahren gemäß einem der Ansprüche 1 bis 8, in dem das Signal durch Oberflächenplasmonresonanz in Echtzeit gemessen wird.

10. Verfahren gemäß einem der Ansprüche 1 bis 9, in dem das messbare Signal gemäß der Erfindung nicht von einem anderen Mediator oder einem Marker, der in dem Medium vorliegt oder dem Medium zugesetzt wurde, als dem Liganden und dem Sensor gemäß der Erfindung stammt.

11. Verfahren gemäß einem der Ansprüche 1 bis 10, in dem man, wenn die Probe einen Mikroorganismus umfasst, außerdem die Menge an Mikroorganismen, die in der Probe zu Beginn der Kultur vorhanden sind, aus der Entwicklung des Wertes des ersten Signals als Funktion der Kulturdauer bestimmt.

12. Verfahren gemäß einem der Ansprüche 1 bis 11, in dem man, wenn die Probe einen Mikroorganismus umfasst, die Sensibilität des Mikroorganismus gegenüber wenigstens einem antimikrobiellen Mittel aus der Entwicklung des Wertes des ersten Signals als Funktion der Kulturdauer nach Einführung des antimikrobiellen Mittels in die Kultur bestimmt.

13. Verfahren gemäß einem der Ansprüche 1 bis 11, in dem man das Vorliegen oder die Menge von mehreren unterschiedlichen Mikroorganismen in der Probe bestimmt.

14. Vorrichtung, die an die Durchführung eines Verfahrens, wie es in einem der Ansprüche 1 bis 13 definiert ist, angepasst ist, umfassend wenigstens eine Kammer, die für die Kultur eines Mikroorganismus in flüssigem Medium angepasst ist, wobei die Kammer wenigstens einen spezifischen Liganden des Mikroorganismus und wenigstens einen nicht-spezifischen Sensor des Mikroorganismus umfasst, die Bindung des Mikroorganismus an dem Liganden ein erstes messbares Signal produziert und die Bindung des Mikroorganismus an dem Sensor ein zweites messbares Signal produziert, der Ligand und der Sensor an einem Träger derart fixiert sind, dass sie mit dem flüssigen Kulturmedium in Kontakt sind.

15. Vorrichtung gemäß Anspruch 14, die in einem Behälter aufgenommen ist, der dazu bestimmt ist, Flüssigkeiten zu sammeln, von denen vermutet wird, dass sie Mikroorganismen enthalten, und/oder die Mikroorganismen zu kultivieren.

## Claims

1. A method for detecting at least one microorganism present in a sample, comprising:
- culturing the sample in a liquid medium in the presence of at least one ligand specific for the microorganism and at least one sensor that is not specific for the microorganism, the binding of said microorganism to the ligand producing a first measurable signal and the binding of said microorganism to the sensor producing a second measurable signal;
- determining values of the first and second signals for at least one culture time;
wherein it is deduced that the sample comprises the microorganism when the values of the first signal and of the second signal are different for the same culture time.

2. The detection method as claimed in claim 1, wherein the culture time is at least twice the generation time of the microorganism.

3. The detection method as claimed in claim 1 or 2, wherein the microorganism is selected from the group consisting of a bacterium, a fungus, an alga, a protozoan, and an isolated cell of a metazoan.

4. The method as claimed in one of claims 1 to 3, wherein the ligand is selected from the group consisting of an antibody, an antibody fragment, an scFv, an aptamer (DNA, RNA), a protein or a glycoprotein, whole bacteriophages or viruses, which are optionally inactivated, bacteriophage or virus fragments, and bacteriophage or virus proteins.

5. The method as claimed in one of claims 1 to 4, wherein the sample is selected from the group consisting of a biological sample, a food sample, a water sample, a soil sample and an air sample.

6. The method as claimed in one of claims 1 to 5, wherein the ligand and/or the sensor are attached to a support for transduction of the signal produced by the binding of a compound to the ligand and/or to the sensor.

7. The method as claimed in one of claims 1 to 6, wherein the first and second signals are measured in real time.

8. The method as claimed in one of claims 1 to 7, wherein the signal is measured by microscopy, by surface plasmon resonance, by resonant mirror, by impedance measurement, by quartz microbalance, by flexible beam, by measurement of light absorption, or by measurement of fluorescence of fluorescent microorganisms.

9. The method as claimed in one of claims 1 to 8, wherein the signal is measured in real time by surface plasmon resonance.

10. The method as claimed in one of claims 1 to 9, wherein the measurable signal according to the invention does not come from a mediator or from a marker present in the medium, or added to the medium, other than the ligand and the sensor according to the invention.

11. The method as claimed in one of claims 1 to 10, wherein, when the sample comprises a microorganism, the amount of microorganisms present in the sample at the beginning of the culture is also determined from the change in the value of the first signal as a function of the culture time.

12. The method as claimed in one of claims 1 to 11, wherein, when the sample comprises a microorganism, the sensitivity of the microorganism to at least one antimicrobial is determined from the change in the value of the first signal as a function of the culture time after introduction of the antimicrobial into the culture.

13. The method as claimed in one of claims 1 to 11, wherein the presence or the amount of several different microorganisms in the sample is determined.

14. A device suitable for carrying out a method as defined in one of claims 1 to 13, comprising at least one chamber suitable for culturing a microorganism in a liquid medium, the chamber comprising at least one ligand specific for the microorganism and at least one sensor that is not specific for the microorganism, the binding of a compound to the ligand producing a first measurable signal and the binding of a compound to the sensor producing a second measurable signal, the ligand and the sensor being attached to a support so as to be in contact with the liquid culture medium.

15. The device as claimed in claim 14, comprised in a container intended for collecting liquids capable of containing microorganisms and/or for culturing microorganisms.
